# EUROPEAN PATENT SPECIFICATION

(11) **EP 0 576 002 B1**
(45) Date of publication and mention of the grant of the patent: **12.03.1997**
(21) Application number: 93110099.4
(22) Date of filing: 24.06.1993
(51) Int. Cl.: C07C 69/757, C12P 7/62

(54) **Optically active 2-alkoxy-carbonyl-2-cycloalkene derivatives and a process for producing the derivatives**
Optisch aktive 2-Alkoxycarbonyl-2-cycloalkene und Verfahren zur Herstellung dieser Derivate
Dérivés du 2-alcoxycarbonyl-2-cycloalcène optiquement actifs et procédé pour produire ces dérivés

(30) Priority: 25.06.1992 JP 190215/92; 25.06.1992 JP 190216/92
(43) Date of publication of application: 29.12.1993
(73) Proprietor: CHISSO CORPORATION, Osaka-shi Osaka 530 (JP)
(72) Inventor: Takano, Seiichi, Sendai-shi, Miyagi-ken (JP); Ogasawara, Kunio, Sendai-shi, Miyagi-ken (JP)
(74) Representative: Hansen, Bernd, Dr. Dipl.-Chem.

(56) References cited:
- EP-A- 0 414 453
- TETRAHEDRON: ASYMMETRY vol. 3, no. 7, July 1992, OXFORD GB pages 837 - 840 SEIICHI TAKANO ET AL. 'Enatiocomplementary Synthesis of Functionalized Cycloalcenol Building Blocks Using Lipase'

## Description

The present invention relates to optically active 2-alkoxycarbonyl-2-cycloalkene derivatives, especially optically active 2-alkoxycarbonyl-2-cycloalkene-1-ols, useful as starting materials of pharmaceuticals, agricultural chemicals and the like, and optically active 2-alkoxycarbonyl-1-acyloxy-2-cycloalkene, and a process for production of these compounds.

The optically active 2-alkoxycarbonyl-2-cycloalkene-1-ols are useful as starting materials for physiologically active compounds such as pharmaceuticals and agricultural chemicals. Namely, the compounds have two different functional groups, a hydroxy group and a carboxyl group, and the compounds are convertible into antipodes each other by isomerization to obtain only one optical isomer. However, since the optically active compounds represented by the following formula (1) or (2) of the present invention have not been synthesized hitherto, it is impossible to satisfactorily value the utility.

In recent years, though methods for production of several kinds of optically active compounds with enzymes by optical resolution have been reported (for example, USP 05107039), the process for producing the compound (1) or (2) of the present invention from the compound represented by the following formula (3) or (4) has not been known

Considering these problems, the inventors of the present invention investigated to obtain two enantiomers of optically active 2-alkoxycarbonyl-2-cycloalkene derivatives. Then, they have found the process for producing these compounds in the presence of lipase.

The first invention provides an optically active 2-alkoxycarbonyl-2-acyloxy-2-cycloalkene represented by the formula (1): 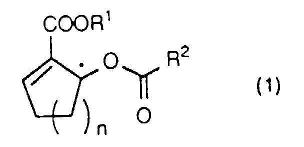 wherein * shows an asymmetric carbon, R¹ and R² are alkyl and n is 1 or 2.

The second invention provides a method for producing an optically active 2-alkoxycarbonyl-2-cycloalkene derivative characterized in that it comprises reacting by transesterification a racemic-2-alkoxycarbonyl-2-cycloalkene-1-ol represented by the general formula (3): 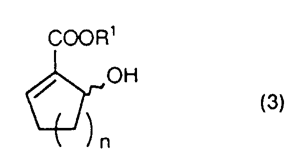 wherein R¹ is alkyl and n is 1 or 2, with an acylating agent in the presence of lipase, and obtaining an optically active 2-alkoxycarbonyl-2-cycloalkene-1-ol represented by the general formula (2): 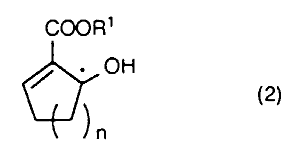 wherein * shows an asymmetric carbon, R¹ is alkyl, and n is 1 or 2, and an optically active 2-alkoxycarbonyl-1-acyloxy-2-cycloalkene represented by the general formula (1): 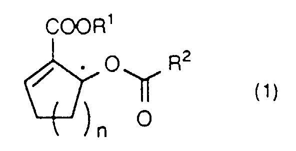 wherein * shows an asymmetric carbon, R¹ and R² are alkyl, and n is 1 or 2, respectively.

The third invention provides a process for producing an optically active 2-alkoxycarbonyl-2-cycloalkene derivative characterized in that it comprises hydrolyzing a racemic-2-alkoxycarbonyl-1-acyloxy-2-cycloalkene represented by the general formula (4): 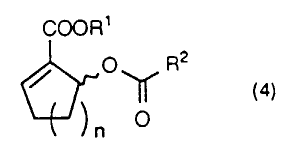 wherein R¹ and R² are alkyl and n is 1 or 2, in the presence of lipase, and obtaining an optically active 2-alkoxycarbonyl-2-cycloalkene-1-ol represented by the general formula (2): 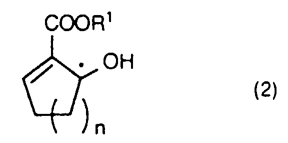 wherein * shows an asymmetric carbon, R¹ is alkyl, and n is 1 or 2, and an optically active 2-alkoxycarbonyl-1-acyloxy-2-cycloalkene represented by the general formula (1): 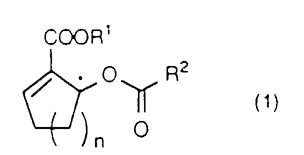 wherein * shows an asymmetric carbon, R¹ and R² are alkyl, and n is 1 or 2, respectively.

The reaction process of the present invention may be represented by the following formulas:

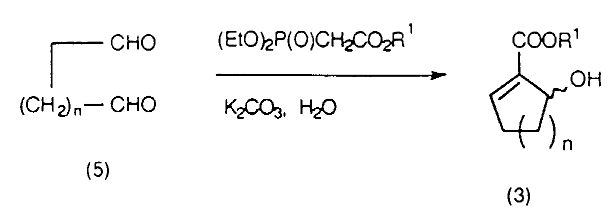

Dialdehyde having formula (5) wherein n=1 as an example, which is a starting material of the process, can be obtained by acid treatment of commercially available 2,5-dimethoxytetrahydrofuran. In the case of n=2, the compound is easily available by oxidation of 1,6-hexanediol or by reduction of adipic acid. When the compound is changed to a racemic-2-alkoxycarbonyl-2-cycloalkene-1-ol, it is easily obtained by a Wittig-Horner reaction (M. Graff, A. Al Dilamani, P. Seguineau, M. Rambaud, J. Villieras, Tetrahedron Lett., 24, 1577 (1986)).

The second process of the present invention is as follows.

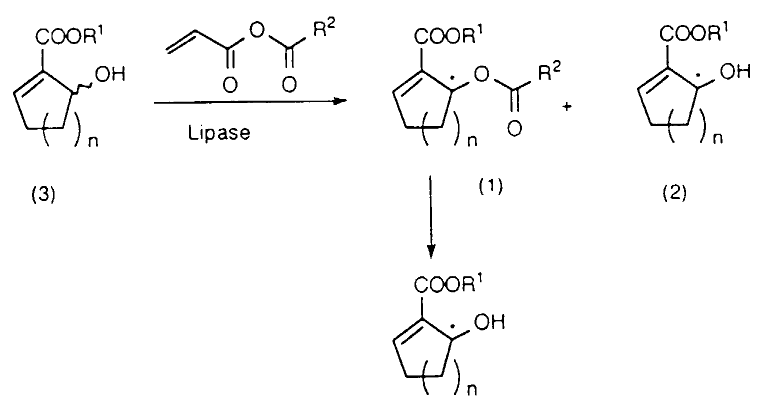

Obtained racemic-2-alkoxycarbonyl-2-cycloalkene-1-ol (3) may be added to a suspension of an acylating agent and lipase, the mixture is stirred at room temperature to preferentially acylate either of the enantiomorphs, and an optically active 2-alkoxycarbonyl-2-cycloalkene-1-ol (2) and an optically active 2-alkoxycarbonyl-1-acyloxy -2-cycloalkene (1) are obtained. Though the reaction time may be varied by loadings, it is preferably 0.3-5 days. For separating these products, for example, column chromatography may be used.

Optically active 2-alkoxycarbonyl-2-cycloalkene-1-ols may be obtained by hydrolyzing optically active 2-alkoxycarbonyl-1-acyloxy-2-cycloalkenes in a phosphoric acid buffer solution (buffer/acetone = 9/1 (V/V)) with lipase. The pH value of the phosphoric acid buffer solution, depending on the kind of lipase, is 4-9, preferably 7-8.

In the above process, fatty acid esters, benzoates and the like which can act as a substrate of transesterification by lipase can be used as the acylating agent, and vinyl esters such as vinyl acetate, vinyl propionate, vinyl butylate, vinyl valerate, vinyl caproate and vinyl laurate can be preferably used. Organic solvents such as benzene, toluene, dichloromethane and t-butyl methyl ether are preferably used. However, if the reaction is not inhibited, any kinds of organic solvents may be used.

As the lipase, if it acts as a catalyst, any kinds of lipase may be used. As microorganisms, the genera Pseudomonas, Arthrobacter, Alcaligenes, Aspergillus, Chromobacterium, Candida, Mucor, Rhizopus, etc, can be exemplified. The lipase may be extracted from the viscera of animals, for example, the liver or the pancreas of pigs or cattle. Particularly, lipase from a Pseudomonas genus can be preferably used. As commercially available lipase, lipase PS, OF, AY and AK (manufactured by Amano Pharmaceutical Co., Ltd.), MY (manufactured by Meito Sangyo) and PPL (manufactured by Sigma company) can be exemplified, and PS is desirable.

As carriers immobilizing the lipase, chromosorb, Cerite (Trade name of Johns-Manville), cellulose, carrageenan or various synthetic polymers by which the lipase activity is not inhibited can be used.

The third process of the present invention is as follows.

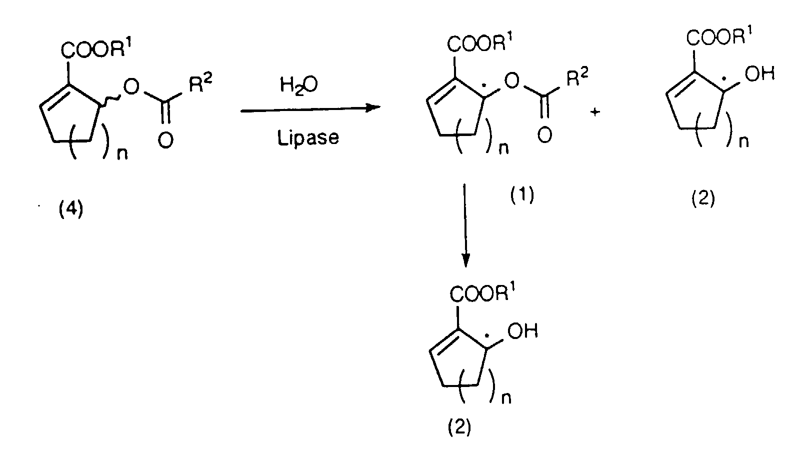

The racemic-2-alkoxycarbonyl-2-cycloalkene-1-ol which can be used as a starting material may be acylated by a conventional method (for example, by treating with acetic anhydride in the presence of pyridine) to obtain a racemic-2-alkoxycarbonyl-1-acyloxy-2-cycloalkene (4).

Then, an optically active 2-alkoxycarbonyl-2-cycloalkene-1-ol (2) and an optically active 2-alkoxycarbonyl-1-acyloxy-2-cycloalkene (3) may be obtained by the process comprising adding the racemic-2-alkoxycarbonyl-1-acyloxy-2-cycloalkene (4) into a lipase suspending buffer solution of phosphoric acid (buffer/acetone = 9/1 (V/V)), and hydrolyzing the mixture. The pH value of the phosphoric acid buffer depends on the lipase, and it is 4-9, preferably 7-8.

The posttreatment is conducted by the same method as in the process of the second invention. Namely, an optically active 2-alkoxycarbonyl-2-cycloalkene-1-ol may be obtained by hydrolyzing an optically active 2-alkoxycarbonyl-1-acyloxy-2-cycloalkene in a phosphoric acid buffer solution (buffer/acetone = 9/1 (V/V)) with lipase or by conventional chemical hydrolysis (for example, stirring in a water-alcohol solution of sodium hydroxide). The pH value of the phosphoric acid buffer, depending on the kind of lipase, is 4-9, preferably 7-8.

As the lipase, if it acts as a catalyst of a stereoselective hydrolysis reaction, any kinds of lipase may be used. As microorganisms, the genera Pseudomonas, Arthrobacter, Alcaligenes, Aspergillus, Chromobacterium, Candida, Mucor, Rhizopus, etc, can be exemplified. The lipase may be extracted from the viscera of animals, for example, the liver or the pancreas of pigs or cattle. Particularly, lipase from a Pseudomonas genus can be preferably used. As commercially available lipase, lipase PS, OF, AY and AK (manufactured by Amano Pharmaceutical Co., Ltd.), MY (manufactured by Meito Sangyo) and PPL (manufactured by Sigma company) can be exemplified, and PS is desirable. When the lipase is immobilized, as carriers immobilizing the lipase, chromosorb, Cerite, cellulose, carrageenan or various synthetic polymers by which the lipase activity is not inhibited can be used.

By the above described process, the optically active 2-alkoxycarbonyl-2-cycloalkene-1-ols can be obtained. By the obtention of the compounds, for example, it becomes possible to produce jasmonate or an intermediate for synthesizing vitamin D₃ useful to a therapeutic drug of osteoporosis by the following reaction process.

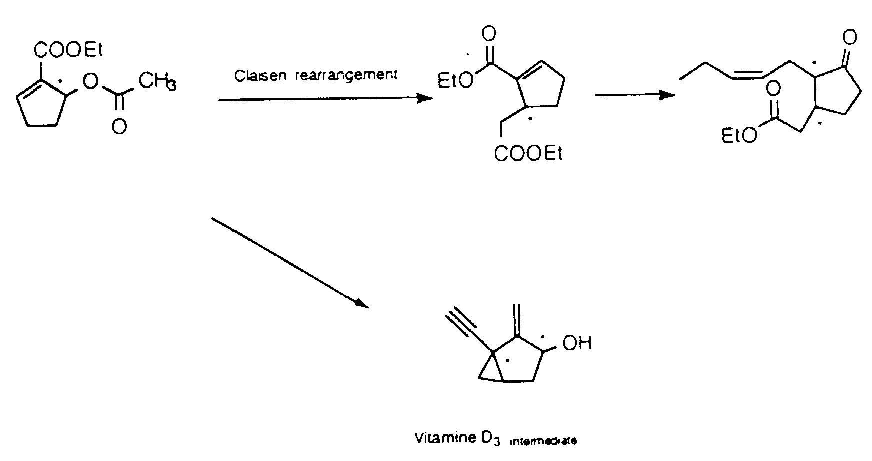

The following examples illustrate this invention more specifically, but these will not always be precise in practical applications.

### Example 1

### Synthesis of (±)-2-ethoxycarbonyl-2-cyclopenten-1-ol

To 26.53 g (201 mmol) of 2,5-dimethoxytetrahydrofuran, 160 ml of 0.6 N hydrochloric acid was added, and the mixture was stirred at 70°C for two hours. After cooling to room temperature, the mixture was neutralized with an aqueous solution of 10% potassium bicarbonate, 1 ml of an aqueous solution of 6 M potassium carbonate was added, and the mixture was stirred at room temperature for 30 minutes. Then, 45.0 g (201 mmol) of triethyl phosphonoacetate was added, 67 ml of an aqueous solution of 6 M potassium carbonate was added dropwise for 20 minutes, and the mixture was stirred for 43 hours. The reaction mixture was extracted with ether, and the organic layer was washed with a saturated sodium chloride acueous solution. After the solution was dried over magnesium sulfate, the solvent was distilled away. 14.3 g (yield 46%) of colorless liquid was obtained by distillation under reduced pressure. Boiling point 90-100°C (0.5 mmHg).

### Synthesis of optically active-2-ethoxycarbonyl-2-cyclopentene-1-ol

To 66 ml of t-butyl methyl ether, 1.027 g (6.58 mmol) of (±)-2-ethoxycarbonyl-2-cyclopentene-1-ol, 1.3 ml (14.1 mmol) of vinyl acetate and 66 mg of lipase PS (produced by Amano Pharmaceutical Co., Ltd.) were suspended. The mixture was stirred at room temperature for 27 hours. The lipase was filtered off with Cerite, the filtrate was concentrated under reduced pressure. The residue was subjected to a column chromatograph over silica gel to separate the desired alcohol and acyl compounds, respectively.

(+)-2-ethoxycarbonyl-1-acetyloxy-2-cyclopentene: 623.5 mg (yield: 47.9%, 100%ee), [α]_{D}³¹ +3.23° (c1.456, CHCl₃), boiling point 45°C (0.1 mmHg)

(-)-2-ethoxycarbonyl-2-cyclopentene-1-ol: 484.3 mg (yield: 47.1%, 99.3%ee), [α]_{D}³¹ -33.52° (c1.256, CHCl₃), boiling point 45°C (1 mmHg).

The optical purity was determined with CHIRAL CEL OD (manufactured by Daicel Co., Ltd., trade name).

### Examples 2-6

The same method as in Example 1 was used, and (±)-2-ethoxycarbonyl-2-cyclopentene-1-ol was used as a starting material. The results are shown in Table 1. Each lipase manufactured by Amano Pharmaceutical Co., Ltd. was used. The reaction time of each example was 72 hours, and a mixture of 1 mmol of the (±)-compound, 2 mmol of vinyl acetate, 10 ml of solvent and 100 mg of lipase were used.

**Table 1**

| Example | Solvent | Alcohol | | Acyl | | Lipase |
|---|---|---|---|---|---|---|
| | | Yield | %ee | Yield | %ee | |
| 2 | CH₂Cl₂ | 22.4 | 96.5 | 53.2 | >99 | PS |
| 3 | benzene | 17.4 | >99 | 53.6 | 92.7 | PS |
| 4 | t-BuOMe | 27.6 | >99 | 43.6 | 82.9 | PS |
| 5 | t-BuOMe | 32.5 | >99 | 42.5 | 88.5 | AK |
| 6 | t-BuOMe | 45.4 | 98.9 | 40.0 | >99 | PPL |

### Example 7

### Hydrolysis of (+)-2-ethoxycarbonyl-1-acetyloxy-2-cyclopentene

To 30 ml of phosphoric acid buffer containing 10% (V/V) acetone, 623.5 mg (3.147 mmol) of (+)-2-ethoxycarbonyl-1-acetyloxy-2-cyclopentene was mixed. After adding 30 mg of lipase PS, the mixture was shaken at 27°C for 24 hours. The reaction solution was filtered on Cerite, and the filtrate was extracted with ether. The organic layer was dried over magnesium sulfate, and the solvent was distilled away under reduced pressure. The residue was subjected to a chromatograph over silica gel to obtain 442 mg (yield 90%) of colorless oil of (+)-2-ethoxycarbonyl-2-cyclopentene-1-ol.

[α]_{D}³⁰ + 34.2° (c1.43, CHCl₃),

### Example 8

### Synthesis of optically active-2-ethoxycarbonylcyclopenta-2-en-1-ol

To 5 ml of phosphoric acid buffer containing 10% (V/V) acetone, 107 mg (0.54mmol) of (±)-2-ethoxycarbonyl-1-acetyloxy-2-cyclopentene was mixed. After adding 11 mg of lipase PS (produced by Amano Pharmaceutical Co., Ltd.), the mixture was shaken at 27°C for 24 hours. The lipase was filtered off with Cerite, the filtrate was extracted with ether, and the organic layer was dried over magnesium sulfate. After the solvent was distilled away under reduced pressure. The residue was subjected to a chromatograph over silica gel to obtain the desired alcohol and acyl compounds, respectively.

(+)-2-ethoxycarbonylcyclopenta-2-ene-1-ol: 35.7 mg (yield: 42.4%, 100%ee), [α]_{D}³⁰ +34.2° (c0.85, CHCl₃),

(-)-2-ethoxycarbonyl-1-acetyloxy-2-cyclopentene: 49.3 mg (yield: 46.1%, 93.4%ee)

The optical purity was determined with CHIRAL CEL OD (manufactured by Daicel Co., Ltd., trade name).

### Examples 9-11

The same method as in Example 1 was used except that lipase was changed. The results are shown in Table 2.

As the lipase, PS manufactured by Amano Pharmaceutical Co., Ltd., PPL (Wako Junyaku Co., Ltd.), and PLE (Sigma Chemical Co.) were used. All of these were 100 mg/mmol of lipase per (±)-compound.

**Table 2**

| Example | Reaction time(day) | Alcohol | | Acyl | | Lipase |
|---|---|---|---|---|---|---|
| | | Yield | %ee | Yield | %ee | |
| 9 | 1 | 46.8 | 100 | 44.8 | 100 | PS |
| 10 | 1 | 28.8 | 74.9 | 18.6 | 73.4 | PPL |
| 11 | 1 | 27.2 | 93.3 | 60.6 | 41.3 | PLE |

### Example 12

### Hydrolysis of (±)-2-ethoxycarbonyl-1-acetyloxy-2-cyclohexene

To 10 ml of phosphoric acid buffer containing 10% (V/V) acetone, 210 mg (0.99 mmol) of (±)-2-ethoxycarbonyl-1-acetyloxy-2-cyclohexene was mixed. After adding 200 mg of lipase PS (manufactured by Amano Pharmaceutical Co., Ltd.), the mixture was shaken at 27°C for 72 hours. The lipase was filtered off with Cerite, the filtrate was extracted with ether, and the organic layer was dried over magnesium sulfate. After the solvent was distilled away under reduced pressure. The residue was subjected to a chromatograph over silica gel to obtain the desired alcohol and acyl compounds, respectively.

(+)-2-ethoxycarbonylcyclohexa-2-ene-1-ol: 71.7 mg (yield: 42.6%, 100%ee), [α]_{D}²⁸ + 57.6° (c0.59, CHCl₃),

(-)-2-ethoxycarbonyl-1-acetyloxy-2-cyclohexene: 33.3 mg (yield: 43.6%, 99.0%ee)

The optical purity was determined with CHIRAL CEL OD (manufactured by Daicel Co., Ltd., trade name).

## Claims

1. An optically active 2-alkoxycarbonyl-1-acyloxy-2-cycloalkene represented by the formula (1): 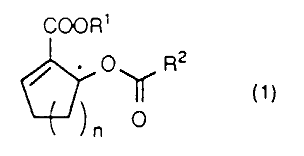 wherein * shows an asymmetric carbon, R¹ and R² are alkyl, and n is 1 or 2.

2. An optically active 2-alkoxycarbonyl-2-cycloalkene-1-ol represented by the formula (2): 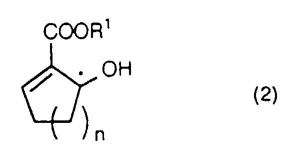 wherein * shows an asymmetric carbon, R¹ and R² are alkyl, and n is 1 or 2.

3. A process for producing an optically active 2-alkoxycarbonyl-2-cycloalkene derivative characterized in that it comprises reacting by transesterisfication a racemic-2-alkoxycarbonyl-2-cycloalkene-1-ol represented by the formula (3): 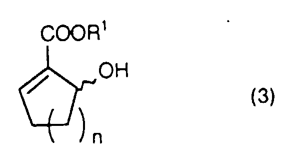 wherein R¹ is alkyl and n is 1 or 2, with an acylating agent in the presence of lipase, and obtaining an optically active 2-alkoxycarbonyl-2-cycloalkenel-1-ol represented by the general formula (2): 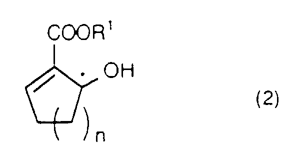 wherein * shows an asymmetric carbon, R¹ is alkyl, and n is 1 or 2, and an optically active 2-alkoxycarbonyl-1-acyloxy-2-cycloalkene represented by the general formula (1): 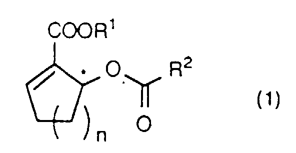 wherein * shows an asymmetric carbon, R¹ and R² are alkyl, and n is 1 or 2, respectively.

4. A process for producing an optically active 2-alkoxycarbonyl-2-cycloalkene-1-ol, said process comprising hydrolyzing in the presence of lipase an optically active 2-alkoxycarbonyl-1-acyloxy-2-cycloalkene represented by the general formula (1) obtained by the process of claim 3.

5. A process for producing an optically active 2-alkoxycarbonyl-2-cycloalkene derivative as claimed in claim 3, wherein R¹ is ethyl.

6. A process for producing an optically active 2-alkoxycarbonyl-2-cycloalkene derivative as claimed in claim 3, wherein R¹ is ethyl and R² is methyl.

7. A process for producing an optically active 2-alkoxycarbonyl-2-cycloalkene derivative as claimed in claim 3, wherein the acylating agent is a vinyl ester.

8. A process for producing an optically active 2-alkoxycarbonyl-2-cycloalkene derivative as claimed in claim 3, wherein the lipase is derived from Pseudomonas.

9. A process for producing an optically active 2-alkoxycarbonyl-2-cycloalkene derivative characterized in that it comprises hydrolyzing a racemic-2-alkoxycarbonyl -1-acyloxy-2-cycloalkene represented by the general formula (4): 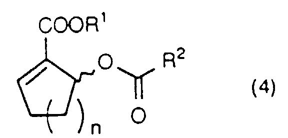 wherein R¹ and R² are alkyl and n is 1 or 2, in the presence of lipase, and obtaining an optically active 2-alkoxycarbonyl-2-cycloalkene-1-ol represented by the general formula (2): 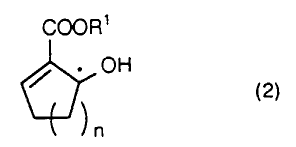 wherein * shows an asymmetric carbon, R¹ is alkyl, and n is 1 or 2, and an optically active 2-alkoxycarbonyl-1-acyloxy-2-cycloalkene represented by the general formula (1): 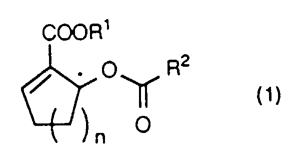 wherein * shows an asymmetric carbon, R¹ and R² are alkyl, and n is 1 or 2, respectively.

10. A process for producing an optically active 2-alkoxycarbonyl-2-cycloalkene-1-ol, said process comprising hydrolyzing an optically active 2-alkoxycarbonyl-1-acyloxy-2-cycloalkene represented by the general formula (1) obtained by the process of claim 9 in the presence of a base.

11. A process for producing an optically active 2-alkoxycarbonyl-2-cycloalkene derivative as claimed in claim 9, wherein R¹ is ethyl.

12. A process for producing an optically active 2-alkoxycarbonyl-2-cycloalkene derivative as claimed in claim 9, wherein R¹ is ethyl and R² is methyl.

13. A process for producing an optically active 2-alkoxycarbonyl-2-cycloalkene derivative as claimed in claim 9, wherein the lipase is derived from Pseudomonas.

## Patentansprüche

1. Optisch aktives 2-Alkoxycarbonyl-l-acyloxy-2-cycloalken, welches durch die Formel (1) dargestellt wird: 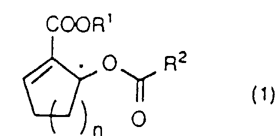 in der * ein asymmetrisches Kohlenstoffatom zeigt, R¹ und R² Alkyl sind und n 1 oder 2 ist.

2. Optisch aktives 2-Alkoxycarbonyl-2-cycloalken-l-ol, welches durch die Formel (2) dargestellt wird: 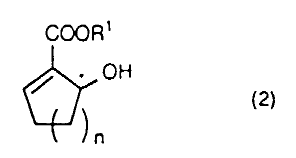 in der * ein asymmetrisches Kohlenstoffatom zeigt, R¹ und R² Alkyl sind und n 1 oder 2 ist.

3. Verfahren zur Herstellung eines optisch aktiven 2-Alkoxycarbonyl-2-cycloalken-Derivats, das dadurch gekennzeichnet ist, daß es die Umsetzung durch Umesterung eines racemischen 2-Alkoxycarbonyl-2-cycloalken-1-ols, das durch die allgemeine Formel (3) dargestellt wird: 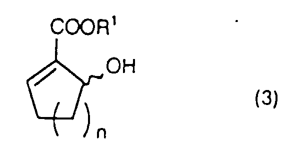 in der R¹ Alkyl und n 1 oder 2 ist, mit einem Acylierungsmittel in Gegenwart von Lipase umfaßt unter Erhalt eines optisch aktiven 2-Alkoxycarbonyl-2-cycloalken-l-ols, welches durch die allgemeine Formel (2) dargestellt wird: 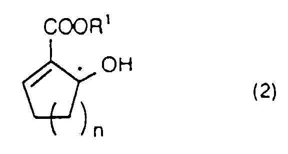 in der * ein asymmetrisches Kohlenstoffatom zeigt, R¹ Alkyl ist und n 1 oder 2 ist, und eines optisch aktiven 2-Alkoxycarbonyl-l-acyloxy-2-cycloalkens, welches durch die allgemeine Formel (1) dargestellt wird: 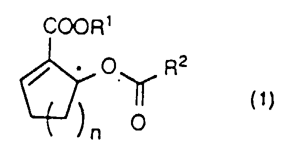 in der * ein asymmetrisches Kohlenstoffatom zeigt, R¹ und R² Alkyl sind und n beziehungsweise 1 oder 2 ist.

4. Verfahren zur Herstellung eines optisch aktiven 2-Alkoxycarbonyl-2-cycloalken-l-ols, dadurch gekennzeichnet, daß besagtes Verfahren die Hydrolyse, in Gegenwart von Lipase, eines optisch aktiven 2-Alkoxycarbonyl-1-acyloxy-2-cycloalkens, dargestellt durch die allgemeine Formel (1) und erhalten durch das Verfahren von Anspruch 3, umfaßt.

5. Verfahren zur Herstellung eines optisch aktiven 2-Alkoxycarbonyl-2-cycloalken Derivats, wie in Anspruch 3 beansprucht, in dem R¹ Ethyl ist.

6. Verfahren zur Herstellung eines optisch aktiven 2-Alkoxycarbonyl-2-cycloalken Derivats, wie in Anspruch 3 beansprucht, in dem R¹ Ethyl und R² Methyl ist.

7. Verfahren zur Herstellung eines optisch aktiven 2-Alkoxycarbonyl-2-cycloalken Derivats, wie in Anspruch 3 beansprucht, in dem das Acylierungsmittel ein Vinylester ist.

8. Verfahren zur Herstellung eines optisch aktiven 2-Alkoxycarbonyl-2-cycloalken Derivats, wie in Anspruch 3 beansprucht, in dem die Lipase von Pseudomonas abgeleitet ist.

9. Verfahren zur Herstellung eines optisch aktiven 2-Alkoxycarbonyl-2-cycloalken Derivats, dadurch gekennzeichnet, daß es die Hydrolyse eines racemischen 2-Alkoxycarbonyl-1-acyloxy-2-cycloalken, das durch die allgemeine Formel (4) dargestellt wird: 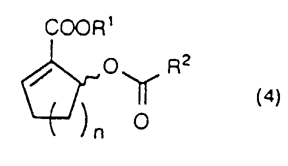 in der R¹ und R² Alkyl sind und n 1 oder 2 ist, in Gegenwart von Lipase, umfaßt unter Erhalt eines optisch aktiven 2-Alkoxycarbonyl-2-cycloalken-l-ols, das durch die allgemeine Formel (2) dargestellt wird: 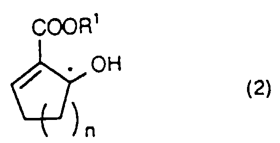 in der * ein asymmetrisches Kohlenstoffatom zeigt, R¹ Alkyl ist und n 1 oder 2 ist und eines optisch aktiven 2-Alkoxycarbonyl-l-acyloxy-2-cycloalkens, das durch die allgemeine Formel (1) dargestellt wird: 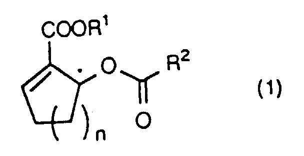 in der * ein asymmetrisches Kohlenstoffatom, R¹ und R² jeweils Alkyl sind und n 1 oder 2 ist,

10. Verfahren zur Herstellung eines optisch aktiven 2-Alkoxycarbonyl-2-cycloalken-1-ols, wobei besagtes Verfahren die Hydrolyse eines optisch aktiven 2-Alkoxycarbonyl-1-acyloxy-2-cycloalkens, das durch die allgemeine Formel (1) dargestellt wird, erhalten durch das Verfahren von Anspruch 9, in der Gegenwart einer Base umfaßt.

11. Verfahren zur Herstellung eines optisch aktiven 2-Alkoxycarbonyl-2-cycloalken Derivats, wie in Anspruch 9 beansprucht, in dem R¹ Ethyl ist.

12. Verfahren zur Herstellung eines optisch aktiven 2-Alkoxycarbonyl-2-cycloalken Derivats, wie in Anspruch 9 beansprucht, in dem R¹ Ethyl und R² Methyl ist.

13. Verfahren zur Herstellung eines optisch aktiven 2-Alkoxycarbonyl-2-cycloalken Derivats, wie in Anspruch 9 beansprucht, in dem die Lipase von Pseudomonas abgeleitet ist.

## Revendications

1. 2-Alcoxycarbonyl-1-acyloxy-2-cycloalcène optiquement actif répondant à la formule (1) : 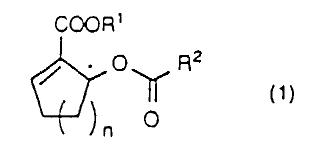 dans laquelle * représente un carbone asymétrique, R¹ et R² sont des groupes alkyle et n est 1 ou 2.

2. 2-Alcoxycarbonyl-2-cycloalcén-1-ol optiquement actif répondant à la formule (2) : 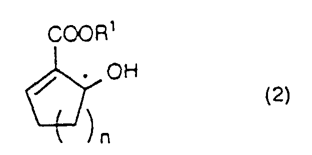 dans laquelle * représente un carbone asymétrique, R¹ et R² sont des groupes alkyle, et n est 1 ou 2.

3. Procédé de préparation d'un dérivé du 2-alcoxycarbonyl-2-cycloalcène optiquement actif, caractérisé en ce qu'il comprend le fait de faire réagir par transestérification un 2-alcoxycarbonyl-2-cycloalcén-1-ol racémique répondant à la formule générale (3) : 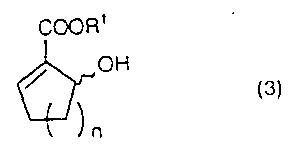 dans laquelle R¹ est un groupe alkyle et n est 1 ou 2, avec un agent d'acylation, en présence de lipase, et d'obtenir un 2-alcoxycarbonyl-2-cycloalcén-1-ol optiquement actif, répondant à la formule générale (2) : 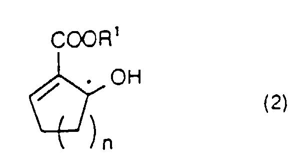 dans laquelle * représente un carbone asymétrique, R¹ est un groupe alkyle et n est 1 ou 2, et un 2-alcoxycarbonyl-1-acyloxy-2-cycloalcène optiquement actif répondant à la formule générale (1) : 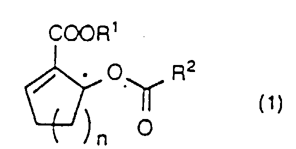 dans laquelle * représente un carbone asymétrique, R¹ et R² sont des groupes alkyle, et n est 1 ou 2, respectivement.

4. Procédé de préparation d'un 2-alcoxycarbonyl-2-cycloalcén-1-ol optiquement actif, ce procédé comprenant le fait d'hydrolyser en présence de lipase un 2-alcoxycarbonyl-1-acyloxy-2-cycloalcène optiquement actif répondant à la formule générale (1) obtenu par le procédé selon la revendication 3.

5. Procédé de préparation d'un dérivé du 2-alcoxycarbonyl-2-cycloalcène optiquement actif tel que revendiqué dans la revendication 3, dans lequel R¹ est un groupe éthyle.

6. Procédé pour la préparation d'un dérivé du 2-alcoxycarbonyl-2-cycloalcène optiquement actif selon la revendication 3, dans lequel R¹ est un groupe éthyle et R² est un groupe méthyle.

7. Procédé de préparation d'un dérivé du 2-alcoxycarbonyl-2-cycloalcène optiquement actif selon la revendication 3, dans lequel l'agent d'acylation est un ester vinylique.

8. Procédé de préparation d'un dérivé du 2-alcoxycarbonyl-2-cycloalcène optiquement actif selon la revendication 3, dans lequel la lipase est obtenue à partir de Pseudomonas.

9. Procédé de préparation d'un dérivé du 2-alcoxycarbonyl-2-cycloalcène optiquement actif, caractérisé en ce qu'il comprend le fait d'hydrolyser un 2-alcoxycarbonyl-1-acyloxy-2-cycloalcène racémique répondant à la formule générale (4) : 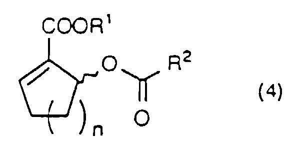 dans laquelle R¹ et R² sont des groupes alkyle et n est 1 ou 2, en présence d'une lipase, et d'obtenir un 2-alcoxycarbonyl-2-cycloalcén-1-ol optiquement actif répondant à la formule générale (2) 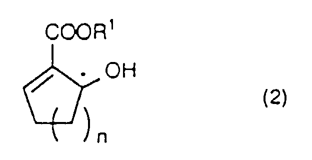 dans laquelle * désigne un carbone asymétrique, R¹ est un groupe alkyle, et n est 1 ou 2, et un 2-alcoxycarbonyl-1-acyloxy-2-cycloalcène optiquement actif, répondant à la formule générale (1) : 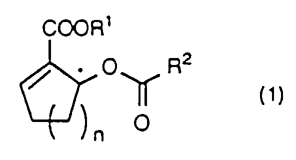 dans laquelle * représente un atome de carbone asymétrique, R¹ et R² sont des groupes alkyle, et n est 1 ou 2, respectivement.

10. Procédé de préparation d'un 2-alcoxycarbonyl-2-cycloalcén-1-ol optiquement actif, ce procédé comprenant le fait d'hydrolyser un 2-alcoxycarbonyl-1-acyloxy-2-cycloalcène optiquement actif répondant à la formule générale (1), obtenu par le procédé selon la revendication 9 en présence d'une base.

11. Procédé de préparation d'un dérivé du 2-alcoxycarbonyl-2-cycloalcène optiquement actif selon la revendication 9, dans lequel R¹ est un groupe éthyle.

12. Procédé pour la préparation d'un dérivé du 2-alcoxycarbonyl-2-cycloalcène optiquement actif selon la revendication 9, dans lequel R¹ est un groupe éthyle et R² est un groupe méthyle.

13. Procédé de préparation d'un dérivé du 2-alcoxycarbonyl-2-cycloalcène optiquement actif selon la revendication 9, dans lequel la lipase est obtenue à partir de Pseudomonas.
